# EUROPEAN PATENT SPECIFICATION

(11) **EP 1 257 280 B1**
(45) Date of publication and mention of the grant of the patent: **05.10.2005**
(21) Application number: 01900579.2
(22) Date of filing: 18.01.2001
(51) Int. Cl.: A61K 31/585, A61P 5/30, A61K 31/565

(54) **PHARMACEUTICAL COMBINATION OF MICRONISED DROSPIRENONE AND AN ESTROGEN FOR HORMONE REPLACEMENT THERAPY**
PHARMAZEUTISCHE KOMBINATION AUS MIKRONISIERTEM DROSPIRENON UND EINEN ESTROGEN FÜR DIE HORMONERSATZTHERAPIE
COMBINAISON PHARMACEUTIQUE DE DROSPIRENONE MICRONISEE ET D'ESTROGENE POUR L'HORMONOTHERAPIE SUBSTITUTIVE

(30) Priority: 18.01.2000 EP 00200183; 18.01.2000 US 484026
(43) Date of publication of application: 20.11.2002
(73) Proprietor: Schering Aktiengesellschaft, 13342 Berlin (DE)
(72) Inventor: HEIL, Wolfgang, 10825 Berlin (DE); HILMANN, Jürgen, 13351 Berlin (DE); LIPP, Ralph, 14169 Berlin (DE); SCHÜRMANN, Rolf, 14167 Berlin (DE)
(74) Representative: Plougmann & Vingtoft A/S
(86) International application number: PCT/IB2001/000041
(87) International publication number: WO 2001/052857

(56) References cited:
- WO-A-95/07081
- WO-A-98/27929

## Description

### FIELD OF INVENTION

The present invention relates to a pharmaceutical composition comprising drospirenone and estrogen, and to methods of hormone replacement therapy by administration of drospirenone and estrogen for estrogen-deficient women.

### GENERAL BACKGROUND OF THE INVENTION

The rise in life expectancy and consequent rise in the number of peri- and post-menopausal women has led to an increase in public and medical awareness of the climacteric period of transition in the reproduction phase of women. Menopause, the last menstruation, occurs between the ages of 45 and 55 in most women. Many factors, including race, genetics, nutrition, altitude, smoking, number of live births, the use of hormonal contraception, length of menstrual cycle and the age of onset of puberty have all been attributed, rightly or wrongly, to affect the age of the last menstrual period.

During these phases of life, female endocrine activity undergoes a series of changes, with the result that the physical and psychological well being of many women is adversely affected. Hormone replacement therapy has aimed to improve the quality of life of women during this natural ageing process to alleviate symptoms associated with this time of transition and to reduce the likelihood or slow the progression of disorders and diseases associated with reduced hormonal activity.

Drospirenone is known from DE 26 52 761 in which its use as a diuretic is disclosed.

The gestagen-like activity of drospirenone and its consequent utility as a contraceptive agent at dosage levels of 0.5-50 mg is disclosed in DE 30 22 337.

The use and role of progestogens in opposed forms of hormone replacement therapy has been studied by the scientific community (Lobo R.A., 1992; Sobel N.B., 1994) as have been regimens comprising estrogens and progestogens (Corson S.L., 1993; Jones K.P., 1992).

The use of a preparation for substitution therapy and for oral contraception comprising at least one progestagen (such as drospirenone in a dose of 0.2-3.0 mg/day) and at least one estrogen (such as estradiol in a dose of 1-2 mg/day), wherein the estrogen dose varies with a periodicity such that blood loss is substantially avoided is disclosed in PCT/EP94/02997 (WO 95 07081). The preparation is provided continuously in an oral form,

The use of a gestagen, such as drospirenone in a dose of 0.5-5 mg/day, in combination with an estrogen, such as estradiol in a dose of 1-3 mg/day for treating disorders, such as sleep disorders, mood changes, nervousness, anxiety, loss of confidence, poor concentration, dimished energy, irritability caused by high testosterone levels is described in WO 98 27929. The combination may be applicable for treating the said symptoms in the peri-menopausal period.

The combination of drospirenone in micronised form (with a surface area more than 10,000 cm²/g and a rapid dissolution of the drospirenone in vitro corresponding to that at least 70% of the active ingredient is dissolved from a solid dosage form over a period of 30 minutes in-vitro dissolution testing) and ethinyl estradiol for inhibiting ovulation in a woman is disclosed in a non pre-published WO 0115701.

Micronised particles are disclosed in Bauer KH, Frömming KH and Führer C (Pharmazeutische Technologle; 4. Aufl.; Thieme, Stuttgart/New York; 1993).

### SUMMARY OF THE INVENTION

The invention relates in a first aspect to a pharmaceutical composition in the form of an oral dosage form comprising
as a first active agent, an estrogen (or naturally or synthetic derivative thereof), with the exception of ethinyl estradiol, in sufficient amounts to treat diseases, disorders and symptoms associated with deficient endogenous levels of estrogen in women, and as a second active agent, 6β,7β;15β;16β-dimethylene-3-oxo-17α-preg-4-ene-21,17-carbolactone (drospirenone) in sufficient amounts to protect the endometrium from the adverse effects of estrogen, together with pharmaceutically acceptable excipients or carriers, said drospirenone is in an amount corresponding to a daily dose ranging from 0.25 to 10 mg and said drospirenone is in micronised form, or said drospirenone is in a form having a surface area of more than 10,000 cm²/g or said drospirenone is in a form having rapid dissolution such that at least 70% of said drospirenone is dissolved within 30 minutes when the composition is subjected to dissolution testing in 900 ml of water at 37°C using USP XXIII Paddle Method II operated at a stirring rate of 50 rpm.

Another aspect of the invention relates to the use of a combination of estrogen and drospirenone for the preparation of a medicament for treating diseases, disorders and symptoms associated with natural menopause, peri-menopause, post-menopause, hypogonadism, castration or primary ovarian failure in women, wherein the amount of estrogen is sufficient to treat diseases, disorders and symptoms associated with deficient endogenous levels of estrogen, said diseases, disorders and symptoms being selected from the group consisting of hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition and osteoporosis and the amount of drospirenone is sufficient to protect the endometrium from the adverse effects of estrogenand said drospirenone is in micronised form, or said drospirenone is in a form having a surface area of more than 10,000 cm²/g or said drospirenone is in a form having rapid dissolution such that at least 70% of said drospirenone is dissolved within 30 minutes when the composition is subjected to dissolution testing in 900 ml of water at 37°C using USP XXIII Paddle Method II operated at a stirring rate of 50 rpm.

In a further aspect, the invention relates to a pharmaceutical preparation consisting of a number of separately packaged and individually removable daily dosage units placed into a packaging unit and intended for oral administration for a period of at least 21 days,
wherein said daily dosage units comprise a combination of estradiol in an amount ranging from about 0.1 to 5 mg and said drospirenone is in micronised form, or said drospirenone is in a form having a surface area of more than 10,000 cm²/g or said drospirenone is in a form having rapid dissolution such that at least 70% of said drospirenone is dissolved within 30 minutes when the composition is subjected to dissolution testing in 900 ml of water at 37°C using USP XXIII Paddle Method II operated at a stirring rate of 50 rpm.

Furthermore, the invention relates to a method of treating and preventing diseases, disorders and symptoms associated with deficient endogenous levels of estrogen in women comprising administering estradiol in amounts corresponding to daily doses of 1 to 3 mg, such as 1, 2 or 3 mg, and drospirenone in amounts corresponding to daily doses of 1 to 3.5 mg, such as 1, 1.5, 2, 2.5, 3, or 3.5 mg.

### DETAILED DISCLOSURE OF THE INVENTION

In the present context, the term cycle itself or when associated with the term menstrual is intended to mean the number of days between menses in a woman. It can range from 21-31 days, typically 28 days.

In the present context, the term menopause is understood as the last natural (ovary - induced) menstruation. It is a single event and a result of an age-dependent dysfunction of the ovarian follicles. Menopause results from the ovaries decreasing their production of the sex hormones estrogen and progesterone. When the number of follicles falls below a certain threshold (a bleeding threshold), the ovaries can no longer produce mature follicles and sex hormones. The ability to reproduce capability ends with menopause.

The peri-menopausal phase begins with the onset of climacteric symptoms when the cycle becomes irregular and ends one year after menopause. The end of peri-menopausal phase can be identified after a protracted period of time without bleeding. Post-menopause is the phase that begins at menopause and continues until death.

One principal aim of hormone replacement therapy is to restore levels of the sex steroid hormones in naturally or prematurely pre-menopausal, menopausal and post-menopausal women or to establish these levels in hypogonadal females.

Monotherapy, also referred to as unopposed therapy, is the treatment with estrogens alone. Exogenous estrogens stimulate the proliferation of the endometrium. In estrogen monotherapy, the opposing effect of progesterone, which terminates proliferation, is absent. The desquamation phase, during which the top layers of the endometrium are shed, does not occur and proliferation of the endometrium occurs to a greater extent than in the phases up to an including the pre-menopausal phase. The result is hyperplasia, a risk factor for endometrial cancer.

Combination therapy, also referred to as opposed therapy, is a treatment where a progestagen is added to protect the endometrium from hyperplasia.

The use of natural progesterone in combination therapy is limited by the low bioavailability of natural progesterone, even in micronized form. Significantly, it has been found that combination therapy comprising the use of drospirenone as a progestagen, is remarkably effective. Drospirenone (DRSP), a 17-α-spirolactone derivative, is a synthetic progestagen that has a surprisingly similar physiological profile to progesterone yet notably better bioavailability. It is the first synthetic progestagen to have a progesterone-like pharmacological profile in that it is antiestrogenic, antiandrogenic, and has antimineralcorticoid activity.

The invention embodies a pharmaceutical composition comprising an estrogen, or naturally or synthetic derivative thereof, in sufficient amounts to treat diseases, disorders and symptoms associated with deficient endogenous levels of estrogen in women, and as a second active agent, 6β,7β;15β;16β-dimethylene-3-oxo-17α-preg-4-ene-21,17-carbolactone (drospirenone) in sufficient amounts to protect the endometrium from the adverse effects of estrogen, together with pharmaceutically acceptable excipients or carriers.

Apart form the active substances themselves, it is envisaged that an ester or prodrug of drospirenone may be employed in the present composition, e.g. an oxyiminopregnane carbolactone as disclosed in WO 98/24801.

Deficient levels of estrogen can occur for a variety of reasons. The composition can be such that it is adequate for deficient levels of estrogen, regardless of the cause. Causes anticipated by the therapy are, but not limited to, natural menopause, peri-menopause, post-menopause, hypogonadism, castration or primary ovarian failure.

Low levels of estrogen, irrespective of the cause, lead to an overall decreased quality of life for women. Symptoms, diseases and disorders range from merely being inconvenient to life threatening. The composition of this therapy anticipates the effective alleviation of all physiological and psychological signs of estrogen deficiency.

Transient symptoms, such as vasomotor signs and psychological symptoms are certainly embodied with the realm of therapy. Vasomotor signs comprise but are not limited to hot flushes, sweating attacks such as night sweats, and palpitations. Psychological symptoms of estrogen deficiency comprise, but are not limited to, insomnia and other sleep disorders, poor memory, loss of confidence, mood changes, anxiety, loss of libido, difficulties in concentration, difficulty in making decisions, diminished energy and drive, irritability, and crying spells.

The treatment of the aforementioned symptoms can be associated with the peri-menopausal phase of a woman's life or after, sometimes long after menopause. It is anticipated that the invention is applicable to these and other transient symptoms during the peri-menopausal phase, menopause, or post-menopausal phase. Moreover, the aforementioned symptoms can be alleviated if the cause of the estrogen deficiency is hypogonadism, castration or primary ovarian failure.

In another embodiment of the invention, the therapy is used for the treatment of permanent effects of estrogen deficiency. Permanent effects comprise physical changes such as urogenital atrophy, atrophy of the breasts, cardiovascular disease, changes in hair distribution, thickness of hair, changes in skin condition and osteoporosis.

Urogenital atrophy, conditions associated with it such as vaginal dryness, increase in vaginal pH and subsequent changes in flora, or events which lead to such atrophy, such as decreases in vascularity, fragmentation of elastic fibres, fusion of collagen fibres, or decreases in cell volume are symptoms thought to be particularly relevant to this therapy. Furthermore, the invention is thought to be relevant to other urogenital changes associated estrogen deficiency such as decreases in the length and/or diameter of the vagina, decreases mucus production, changes in cell population, decreases in glycogen production, decreases in growth of lactobacilli or increases in growth of streptococci, staphylococci, or coliform bacilli. Other associated changes that are thought to be preventable by the invention are those that may render the vagina susceptible to injury or infection, such as exudative discharges, vaginitis, and dyspareunia. Furthermore, infections of the urinary tract and incontinence are other common symptoms associated with lowered estrogen levels.

Other embodiments of the invention include the prevention or alleviation of physical changes associated with estrogen deficiency, such as changes in the skin, changes in hair distribution, thickness of hair, atrophy of the breasts, or osteoporosis.

The prevention and management of osteoporosis, most notably post-menopausal osteoporosis, is a particularly interesting embodiment of the invention. Furthermore, bone demineralisation, reduction of bone mass and density, thinning and interruption of trabeculae, and/or consequent increase in bone fractures or bone deformations are thought to be particularly relevant. The prophylactic treatment of osteoporosis is an interesting therapeutic application of the invention.

A particularly interesting embodiment of the invention comprises the use of the composition for lessening the frequency, persistence, duration and/or severity of hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition and osteoporosis, most notably hot flushes, sweating attacks, palpitations, sleep disorders, mood changes, nervousness, anxiety, urogenital atrophy, atrophy of the breasts or for the prevention or management of osteoporosis.

The pharmaceutical composition for HRT disclosed herein comprises estrogen with the exception of ethinyl estradiol. In preferred embodiments, the estrogen is selected from the group consisting of estradiol, estradiol sulfamates, estradiol valerate, estradiol benzoate, estrone, estriol, estriol succinate and conjugated estrogens, including conjugated equine estrogens such as estrone sulfate, 17β-estradiol sulfate, 17α-estradiol sulfate, equilin sulfate, 17β-dihydroequilin sulfate, 17α-dihydroequilin sulfate, equilenin sulfate, 17β-dihydroequilenin sulfate and17α-dihydroequilenin sulfate or mixtures thereof. Particularly interesting estrogens are selected from the group consisting of estradiol, estradiol sulfamates, estradiol valerate, estradiol benzoate, estrone, and estrone sulfate or mixtures thereof, notably estradiol, estradiol valerate, estradiol benzoate and estradiol sulfamates. Most preferred are estradiol or estradiol sulfamates, particularly estradiol.

Thought to be particularly relevant are micronized forms of estrogens, such as micronized estradiol, micronized estradiol sulfamates, micronized estradiol valerate, micronized estradiol benzoate, micronized estrone, or micronized estrone sulfate or mixtures thereof, notably micronized estradiol, micronized estradiol valerate, micronized estradiol benzoate or micronized estradiol sulfamates. Most preferred is micronized estradiol or micronized estradiol sulfamates, particularly micronized estradiol.

In certain embodiments of the invention, wherein the composition comprises more than one estrogen, one or more estrogen may be in micronized form, such as 2 or all of the estrogen.

Moreover, an interesting embodiment of the invention comprises a composition wherein the drospirenone (DRSP) is in micronized form, such that one or both estrogen and DRSP are in micronized form, preferably both estrogen and DRSP are in micronized form.

Drospirenone, which may be prepared substantially as described in, e.g., US 4,129,564 or WO 98/06738, is a sparingly soluble substance in water and aqueous buffers at various pH values. Furthermore, drospirenone is rearranged to an inactive isomer under acid conditions and hydrolysed under alkaline conditions. To ensure good bioavailability of the compound, it is therefore advantageously provided in a form that promotes rapid dissolution thereof.

It has been found that when drospirenone is provided in micronized form in a pharmaceutical composition, rapid dissolution of the active compound from the composition occurs *in vitro.* A micronized substance is such that a test batch (ca. 200 mg) of the particles, herein drospirenone particles, has a surface area of more than 10,000 cm²/g, and has the following particle size distribution for drospirenone as determined under the microscope: not more than 2 particles in a given batch (ca. 200 mg) are with a diameter of more than 30 µm, and preferably ≤ 20 particles with a diameter of ≥ 10 µm and ≤ 30 µm. The term "rapid dissolution" is defined as the dissolution of at least 70% over about 30 minutes, in particular at least 80% over about 20 minutes, of drospirenone from a tablet preparation containing 3 mg of drospirenone in 900 ml of water at 37°C determined by the USP XXIII Paddle Method using a USP dissolution test apparatus 2 at 50 rpm.

As an alternative to providing the drospirenone in micronized form, it is possible to dissolve it in a suitable solvent, e.g. methanol or ethyl acetate, and spray it onto the surface of inert carrier particles followed by incorporation of the particles containing drospirenone on their surface in the composition.

Without wishing to be limited to any particular theory, it appears that the *in vitro* dissolution rate of drospirenone is connected to the dissolution rate *in vivo* resulting in rapid absorption of drospirenone *in vivo* on oral administration of the compound. This is an advantage because isomerization of the compound in the gastric environment and/or hydrolysis in the intestine is substantially reduced, leading to a high bioavailability of the compound.

With respect to the estrogen which may also be a sparingly soluble substance, though usually less sensitive to degradation than drospirenone under conditions prevailing in the gastrointestinal tract, it is also an advantage to provide it in micronized form or sprayed from a solution, e.g. in ethanol, onto the surface of inert carrier particles. This has the added advantage of facilitating a more homogenous distribution of the estrogen throughout the composition. When the estrogen is provided in micronized form, it preferably has the following particle size distribution as determined under the microscope: 100% of the particles have a diameter of ≤ 15.0 µm, 99% of the particles have a diameter of ≤ 12.5µm, 95% of the particles have a diameter of ≤ 10.0 µm, and 50% of the particles have a diameter of ≤ 3.0 µm. Furthermore, no particle is larger than 20 µm, and ≤ 10 particles have a diameter of ≥ 15 µm and ≤ 20 µm.

The particle size distribution for estradiol or estradiol hemihydrate is preferably such that 100% of the particles in a given batch have a diameter less than 15.0 µm, 99% have a diameter less than 12.5 µm, 95% have a diameter less than 10.0 µm, 50% have a diameter less than 3.0 µm, or 40% have a diameter less than 1.1 µm.

To obtain a more rapid rate of dissolution, it is preferred to include carriers or excipients, which act to promote dissolution of both active substances. Examples of such carriers and excipients include substances that are readily soluble in water such as cellulose derivatives, carboxymethyl cellulose, hydroxypropyl cellulose, hydroxypropyl methyl cellulose, gelled starch, gelatin or polyvinylpyrrolidone. In particular, it is anticipated that polyvinylpyrrolidone might be particularly helpful to promote dissolution.

The dose of the drospirenone in each composition is preferably such as to protect the endometrium from the adverse effects of estrogen. DRSP, in sufficient doses, may be used as an opponent to estrogen to protect the endometrium form hyperplasia or cancer.

In some cases, however, the dose of DRSP may be sufficient so as to stabilise the menstrual cycle and bleeding pattern. In such cases, doses of estrogen may be low or nil. Adipose tissue production of estrogen may be such that no or very low doses of estrogen are required to stabilise the menstrual cycle and bleeding pattern. Moreover, in certain embodiments, where the woman suffers from other disorders not compatible with the use of an exogenous source of estrogen (such as for the absolute contraindications of severe liver diseases and pregnancy or for the relative contraindications endometrial carcinoma and endometriosis, mammary carcinoma, venous thromboembolism, hypertension, diabetes mellitus, otosclerosis and melanoma) no or very low amounts of estrogen may be in the composition. In such embodiments, the dose of DRSP may be such that the disorder, disease, or symptom is alleviated.

In preferred embodiments, the dose of DRSP corresponds to 15 to 70 mg per cycle, such as 20 to 60 mg per cycle, particularly 40 to 60 mg per cycle. The length of the cycle, as stated *supra* may vary from 21to31 days. Viewed otherwise, a composition may comprise of an amount of DRSP corresponding to a daily dose ranging from 0.25 to 10, such as about 0.25 to 8, 0.25 to 6, 0.25 to 5, 0.5 to 4.5, 1 to 4, and 1.5 to 3.5 mg.

The dose of estrogen may vary from woman to woman, depending on the phase of her life (peri-menopausal or post-menopausal), endogenous levels of estrogen, the severity of the symptom(s), disorder or disease, the disorder, disease or symptom targeted, the use by the woman of other medicaments for other purposes, and other pharmacokinetic variables.

In other embodiments, the dose of estrogen and/or DRSP is sufficient to treat hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition or for the prevention or management of osteoporosis.

The dose of estrogen and/or DRSP may depend on the treatment, be it for endometrial protection such as bleeding pattern, for osteoporosis prevention or management, for the treatment of menopausal symptoms, such as the for the reduction in the number, frequency, and severity of hot flushes, night sweats, mood swings palpitations, insomnia and other sleep disorders, mood changes, nervousness, anxiety, poor memory, loss of confidence, loss of libido, poor concentration, diminished energy, diminished drive and irritability.

In embodiments wherein the estrogen is estradiol, the amount of estradiol corresponds to a daily dose ranging from 0.1 to 5 mg, such as about 0.2 to 4.5, 0.5 to 4, 1 to 3, in particular 1, 2, or 3 mg.

With regards to the doses of estradiol derivatives with greater potency, e.g. estradiol valerate, the comparable dosage may be calculated by adjusting the above-mentioned dosages in accordance to the relative potency.

In embodiments where the woman is peri-menopausal the dose of estrogen and/or DRSP may depend on the day of the cycle, that is, whether she is in the preovulatory or postovulatory phase of the cycle, and how advanced within each phase. In embodiments where the woman is post-menopausal or even pre-menopausal, the dose of estrogen and/or DRSP may depend on the time since the last menstruation.

In certain embodiments of the invention, the medicament is administered in the form a of a number of separately packaged and individually removable dosage units placed in a packaging unit and intended for oral administration for a period of at least 21, such as at least 28 days, such as at least 30 or 31 days. In such embodiments, the dose of DRSP and/or estrogen may be the same within each dosage unit or may vary. In embodiments where the amount of DRSP and/or estrogen in the dosage unit varies according to the phase or day within the period of at least 21 days, such as at least 28 days that said dosage unit is to be administered, such compositions, methods of treatments and preparations are termed multi-phased.

The dose proportionality may vary according to its use. In preferred embodiments the dose proportionality of estrogen and drospirenone for the preparation of a medicament is such that estrogen doses are to treat diseases, disorders and symptoms associated with deficient endogenous levels of estrogen and the amount of drospirenone is sufficient to protect the endometrium from the adverse effects of estrogen.

In preferred embodiments, the dose proportionality is sufficient to treat hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition or for the prevention or management of osteoporosis.

The invention relates to a method of treating diseases, disorders and symptoms associated with deficient endogenous levels of estrogen in women comprising administering estrogen in sufficient amounts to alleviate said symptoms and drospirenone in sufficient amounts to protect the endometrium from adverse effects of estrogen. Preferably, the deficient levels of estrogen to which the method applies are caused by natural menopause, peri-menopause, post-menopause, hypogonadism, castration or primary ovarian failure.

The diseases, disorders and symptoms to which the method applies comprise hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition or for the prevention or management of osteoporosis. Specifically, the method is anticipated to apply to hot flushes, sweating attacks, palpitations, sleep disorders, mood changes, nervousness, anxiety, urogenital atrophy, atrophy of the breasts or for the prevention or management of osteoporosis.

The method applies to the administration of an estrogen, preferably estradiol, estradiol sulfamates, estradiol valerate, estradiol benzoate, ethinyl estradiol, estrone, estriol, estriol succinate and conjugated estrogens, including conjugated equine estrogens such as estrone sulfate, 17β-estradiol sulfate, 17α-estradiol sulfate, equilin sulfate, 17β-dihydroequilin sulfate, 17α-dihydroequilin sulfate, equilenin sulfate, 17β-dihydroequilenin sulfate and17α-dihydroequilenin sulfate or mixtures thereof, most estradiol, estradiol sulfamates, estradiol valerate, estradiol benzoate, estrone, and estrone sulfate or mixtures thereof, particularly estradiol.

A particularly attractive embodiment of the invention comprises the administration of drospirenone (DRSP) and/or the estrogen in micronized form. Furthermore, of particular interest is where the estrogen is estradiol in micronized form. In such embodiments, one or both active ingredients are administered in micronized form.

In certain embodiments, the method comprises the administration of a dose of DRSP corresponding to 15 to 70 mg per cycle, such as 20 to 60 mg per cycle, particularly 40 to 60 mg per cycle. The method preferably comprises the administration a dose of DRSP the amount of DRSP corresponding to a daily dose ranging from 0.25 to 10 mg, such as about 0.25 to 8, 0.25 to 6, 0.25 to 5, 0.5 to 4.5, 1 to 4, or 1.5 to 3.5 mg.

The amount of estradiol administered may correspond to a daily dose ranging from 0.1 to 5 mg, such as about 0.2 to 4.5, 0.5 to 4, 1 to 3, in particular 1, 2 or 3 mg.

A particularly pertinent embodiment relates to a pharmaceutical composition comprising, as a first active agent, estradiol in amounts corresponding to a daily dose of 1 to 3 mg to treat diseases, disorders and symptoms associated with deficient endogenous levels of estrogen in women and as a second active agent 6β,7β;15β;16β-dimethylene-3-oxo-17α-preg-4-ene-21,17-carbolactone (drospirenone) in amounts corresponding to a daily dose of 1 to 3.5 mg sufficient amounts to protect the endometrium from the adverse effects of estrogen together with a pharmaceutically acceptable excipient or carrier.

Certain preferred combinations with regards to the active ingredients in the composition, wherein the estrogen is estradiol comprise 1 mg of estradiol with 0.5 mg of DRSP, 1 mg of estradiol with 1 mg of DRSP, 1 mg of estradiol with 1.5 mg of DRSP, 1 mg of estradiol with 2 mg of DRSP, 1 mg of estradiol with 2.5 mg of DRSP, 1 mg of estradiol with 3 mg of DRSP, 2 mg of estradiol with 1 mg of DRSP and 2 mg of estradiol with 4 mg of DRSP.

In a preferred embodiment, a preferred embodiment of the invention relates to a pharmaceutical composition comprising as a first active agent estradiol in amounts corresponding to a daily dose of 1 to 3 mg, such as 1, 1.5, 2, 2.5, or 3 mg of estradiol, and as a second active agent 6β,7β;15β;16β-dimethylene-3-oxo-17α-preg-4-ene-21,17-carbolactone (drospirenone) in amounts corresponding to a daily dose of 1 to 3.5 mg, such as 1, 1.5, 2, 2.5, 3, or 3.5 mg of DRSP, together with a pharmaceutically acceptable excipient or carrier.

Accordingly, a preferred embodiment of the invention relates to a method of treating and preventing diseases, disorders and symptoms associated with deficient endogenous levels of estrogen in women comprising administering estradiol in amounts corresponding to daily doses of 1 to 3 mg, such as 1, 2 or 3 mg, and drospirenone in amounts corresponding to daily doses of 1 to 3.5 mg, such as 1, 1.5, 2, 2.5, 3, or 3.5 mg.

Given that deficient endogenous levels of estrogens may be associated with, amongst other conditions, natural menopause, peri-menopause, post-menopause, hypogonadism, castration or primary ovarian failure, the method of treating and preventing associated diseases, disorders and symptoms may persist until the death of the individual. That is to say that the composition may be administered from the diagnosis of the disease, disorder or symptoms for the entirety of the individual's life. In certain embodiments, the method and the composition may be based on rhythm of the menstrual cycle. In other embodiments, the method may totally disregard the natural cycle.

The method is preferably multi-phased. The method may comprise administering for 10 to 12 days a daily dosage unit comprising estradiol in amounts corresponding to daily doses ranging from 0.1 to 5 mg; and further administering for 10 to 12 days a daily dosage unit comprising estradiol in amounts corresponding to daily doses ranging from 0.1 to 5 mg and drospirenone in amounts corresponding to daily doses ranging from 0.25 to 6 mg; and further administering for 4 to 8 days a daily dosage unit comprising estradiol in amounts corresponding to daily doses ranging from 0.25 to 5 mg.

Similarly, the multi-phased method may comprise administering for 10 to 12 days a daily dosage unit comprising estradiol in amounts corresponding to daily doses ranging from 0.1 to 5 mg; and further administering for 10 to 12 days a daily dosage unit comprising estradiol in amounts corresponding to daily doses ranging from 0.1 to 5 mg and drospirenone in amounts corresponding to daily doses ranging from 0.25 to 6 mg; and further administering for 4 to 8 days a daily dosage unit comprising of a placebo or blank.

A regimen of the method may comprise administering for at least 21 days a daily dosage unit comprising estradiol in amounts corresponding to daily doses ranging from 0.1 to 5 mg and drospirenone in amounts corresponding to daily doses ranging from 0.25 to 6 mg; and further administering for no more than 7 days a daily dosage unit comprising of a placebo or blank. A similar regimen of method may comprise administering for at least 21 days a daily dosage unit comprising estradiol in amounts corresponding to daily doses ranging from 0.1 to 5 mg and drospirenone in amounts corresponding to daily doses ranging from 0.25 to 6 mg; and further administering for no more than 7 days a daily dosage unit comprising estradiol in amounts corresponding to daily doses ranging from 0.1 to 5 mg.

Alternatively, a regimen of the method may comprise administering for at least 21 days a daily dosage unit comprising estradiol in amounts corresponding to daily doses ranging from 0.1 to 5 mg and drospirenone in amounts corresponding to daily doses ranging from 0.25 to 6 mg; and not administering a dosage unit for no more than 7 days.

An alternative embodiment of the method comprises administering for 21 to 28 days a daily dosage unit comprising estradiol in amounts corresponding to daily doses ranging from 0.1 to 5 mg and drospirenone in amounts corresponding to daily doses ranging from 0.25 to 6 mg.

The regimen may comprise of a continuous administration; that is to say that throughout the 21 to 28 days, a daily dose of estrogen is administered. Likewise, drospirenone may administered continuously, such that either one or both of estrogen and DRSP is/are administered continuously.

In another embodiment, estrogen is administered continuously and drospirenone is administered sequentially. In such an embodiment, throughout the continuous administration of estrogen, DRSP is administered at regular intervals, for 1 to 20 days, such as for 3 to 15 days, 5 to 14 days, particularly for 6 to 14 days. In another interesting embodiment of the regimen of the method, the estrogen dosage is lower for the 1 to 7 days immediately following said sequential administration of drospirenone.

Furthermore, in one embodiment of the invention the use of claim 35 for treating and preventing diseases, disorders and symptoms associated with deficient endogenous levels of estrogen in women comprises continuous administration of estrogen and interrupted administration of progestin. Specifically the method may estrogen being administered continuously for 21 to 30 days and drospirenone being administered in a 3-day-on-3-day-off cycle. A particularly attractive embodiment within this alternative drospirenone being administered on days 4 through 6, 10 through 12, 16 through 18, 22 through 24, and 28 through 30, whilst estrogen, such as estradiol is administered continuously.

The composition of the dosage unit may be formulated in any way conventional in the pharmaceutical art. In particular, as indicated above, the composition may be formulated by a method comprising providing drospirenone and, if desired, an estrogen such as estradiol in micronized form in said unit dosage form, or sprayed from a solution onto particles of an inert carrier in admixture with one or more pharmaceutically acceptable excipients that promote dissolution of the drospirenone and an estrogen so as to promote rapid dissolution of drospirenone and preferably estradiol on oral administration. Examples of suitable excipients include fillers, e.g. sugars such as lactose, glucose or sucrose, sugar alcohols such as mannitol, starch such as corn or potato starch or modified starch, lubricants such as talc or magnesium stearate and binders such as polyvinylpyrrolidone, cellulose derivatives, carboxymethyl cellulose, hydroxypropyl cellulose, hydroxypropylmethyl cellulose, methyl cellulose, or gelatin for making oral dosage forms such as tablets, pills or capsules. Tablets may conveniently be coated with a suitable film-forming agent, e.g. hydroxypropylmethylcellulose. The present composition may also be formulated in liquid form, e.g. as solutions, suspensions or emulsions, together with conventional diluents or excipients in a manner known per se in the pharmaceutical art.

A particularly interesting regimen of a multi-phased pharmaceutical preparation comprises the sequential administration of both estrogen and drospirenone. An attractive embodiment of such as regimen will comprise a treatment free interval wherein no dosage unit is administered such as a comprising administering for 20 to 24 days a daily dosage unit comprising estradiol in amounts corresponding to daily doses ranging from 0.1 to 5 mg, and drospirenone in amounts corresponding to daily doses ranging from 0.25 to 6 mg for the last 10 to 12 days of said 20 to 24 day, and not administering any dosage units for 4 to 8 days.

Alternatively, any interval wherein neither DRSP nor estrogen is administered, a placebo or blank is administered. Such a regimen of hormone replacement therapy can effectively comprise a method comprising administering for 20 to 24 days a daily dosage unit comprising estradiol in amounts corresponding to daily doses ranging from 0.1 to 5 mg, and further administering drospirenone in amounts corresponding to daily doses ranging from 0.25 to 6 mg for the last 10 to 12 days of said 20 to 24 day, and further administering for 4 to 8 days a daily dosage unit comprising no active ingredient.

An alternative regimen comprises administering for 20 to 24 days a daily dosage unit comprising estradiol in amounts corresponding to daily doses ranging from 0.1 to 5 mg, and further administering drospirenone in amounts corresponding to daily doses ranging from 0.25 to 6 mg for the last 10 to 12 days of said 20 to 24 day, followed by administering for 4 to 8 days a daily dosage of unit comprising estradiol in amounts less than daily dosage unit taken for said 20 to 24 day administration of estradiol.

In embodiments where the medicament is in the form a of a number of separately packaged and individually removable dosage units placed in a packaging unit and intended for oral administration for a period of at least 21, such as at least 28 days and wherein the estrogen is estradiol, preferably at least 21 daily dosage units comprise a combination of estradiol in an amount from about 0.1 to 5 mg and drospirenone in an amount ranging from about 0.25 to 6 mg; and 7 or less daily dosage units comprise estradiol in an amount from about 0.1 to 5 mg.

Alternatively, the medicament may be in the form of a number of separately packaged and individually removable dosage units placed in a packaging unit and intended for oral administration for a period of at least 28 days and at least 21 daily dosage units comprise a combination of estradiol in an amount from about 0.1 to 5 mg and drospirenone in an amount ranging from about 0.25 to 6 mg and 7 or less daily dosage units comprise a blank or placebo.

It easily follows that the medicament may be in the form of a number of separately packaged and individually removable dosage units placed in a packaging unit and intended for oral administration for a period of at least 28 days and at least 21 daily dosage units comprise a combination of estradiol in an amount from about 0.1 to 5 mg and drospirenone in an amount ranging from about 0.25 to 6 mg. There may not be any dosage units for the last 7 or less days of the regimen.

Patient compliance of the many embodiments of the regimen may be aided by means of a pharmaceutical preparation tailored to the patients needs or habits. One such preparation may consist of a number of separately packaged and individually removable daily dosage units placed into a packaging unit and intended for oral administration for a period of at least 21 days, such as at least 28 days, wherein said daily dosage units comprise a combination of estradiol in an amount ranging from about 0.1 to 5 mg and drospirenone in an amount ranging from about 0.25 to 6 mg.

Furthermore, patient compliance may be aided by a regimen comprising a multi-phase pharmaceutical preparation.

Every regimen may be easily be adhered to by a multi-phased pharmaceutical preparation such as one consisting of a number of separately packaged and individually removable daily dosage units placed into a packaging unit and intended for oral administration for a period of 28 days wherein said daily dosage units comprise a combination of estradiol in an amount ranging from about 0.1 to 5 mg and drospirenone in an amount ranging from about 0.25 to 6 mg. In such a preparation, the amount of active ingredient varies through the 28-day period.

An attractive embodiment relates to multi-phased pharmaceutical preparation consisting of a number of separately packaged and individually removable daily dosage units placed into a packaging unit and intended for oral administration for a period of 21 to 30 consecutive days wherein 10 to 15 said daily dosage units comprise a combination of estradiol in an amount ranging from about 0.1 to 5 mg and drospirenone in an amount ranging from about 0.25 to 6 mg; and 10 to 15 said daily dosage units comprise estradiol in an amount ranging from about 0.1 to 5 mg. This embodiment is particularly suited for preparations wherein estrogen is administered continuously for 21 to 30 days and drospirenone is administered in a 3-day-on-3-day-off cycle. Preferably, within this embodiment, the preparation is designed so that drospirenone is administered on days 4 through 6, 10 through 12, 16 through 18, 22 through 24, and days 28 through 30.

Moreover, a multi-phased pharmaceutical preparation wherein the number of daily dosage units is 21 or 28, or a multiple of 21 or 28, such as 2 to 24, such as 2 to 12, particularly 2 to 8, such as multiples of 2 to 6.

Likewise, the invention relates to a method of treating diseases, disorders, and symptoms associated with estrogen deficiency comprising a multi-phased pharmaceutical preparation comprising daily dosage units which are administered for 1 to 12, preferably 2 to 8, such as 2, 3, 4 5,6, 7, and 8 multiples of 28 days.

A packaging unit comprising the daily dosage units described above may be prepared in a manner analogous to that of making oral contraceptives or hormone replacement regimens. This may for instance be a conventional blister pack or any other form known for this purpose, for instance a pack comprising the appropriate number of dosage units (in this case at least 28, or for particular applications, a multiple of 28) in a sealed blister pack with a cardboard, paperboard, foil or plastic backing and enclosed in a suitable cover. Each blister container may conveniently be numbered or otherwise marked.

It is also envisaged that the present composition may be in the form of a parenteral formulation such as a subcutaneous implant or transdermal formulation. For making implants, the active agents may suitably be formulated together with one or more polymers that are gradually eroded or degraded when in use, e.g. silicone polymers, ethylene vinylacetate, polyethylene or polypropylene.

Where transdermal formulations are concerned, they may be prepared in the form of matrices or membranes or as fluid or viscous formulations in oil or hydrogels. For transdermal patches, an adhesive which is compatible with the skin should be included, such as polyacrylate, a silicone adhesive or polyisobutylene, as well as a foil made of, e.g. polyethylene, polypropylene, ethylene vinylacetate, polyvinylchloride, polyvinylidene chloride or polyester, and a removable protective foil made from, e.g., polyester or paper coated with silicone or a fluoropolymer. For the preparation of transdermal solutions or gels, water or organic solvents or mixtures thereof may be used. Transdermal gels may furthermore contain one or more suitable gelling agents or thickeners such as silicone, tragacanth, starch or starch derivatives, cellulose or cellulose derivatives or polyacrylic acids or derivatives thereof. Transdermal formulations may also suitably contain one or more substances that enhance absorption though the skin, such as bile salts or derivatives thereof and/or phospholipids. Suitable transdermal formulations may, for instance, be made in a manner analogous to that described in WO 94/04157 for 3-ketodesogestrel. Alternatively, transdermal formulations may be prepared according to a method disclosed in, e.g., BW Barry, "Dermatological Formulations, Percutaneous Absorption", Marcel Dekker Inc., New York - Basel, 1983, or YW Chien, "Transdermal Controlled Systemic Medications", Marcel Dekker Inc., New York - Basel, 1987.

The present invention is further described in the following examples.

### EXPERIMENTAL

### Example 1

### Preparation of tablets containing drospirenone and estradiol may be performed in the following manner

| Tablet cores of the following composition | |
|---|---|
| micronized drospirenone | 3.00 mg |
| micronized estradiol | 1.00, 2.00, 3.00 mg |
| lactose monohydrate | 45.2, 46.2, 47.2 mg |
| corn starch | 14.40 mg |
| modified starch | 9.60 mg |
| polyvinylpyrrolidone 25,000 | 4.00 mg |
| magnesium stearate | 0.80 mg |

are prepared by charging a fluidised bed granulator with 31.68 kg corn starch, 21.12 kg modified starch, 6.60 kg micronized drospirenone, 2.20, 4.40 or 6.6 kg of micronized estradiol (for 1 mg, 2 mg, and 3 mg dose, respectively) and 99.44, 101.64, or 103.84 kg of lactose monohydrate (for 3 mg, 2 mg, and 1 mg dose, respectively) and activating the fluidised bed. An aqueous solution of 8.80 kg polyvinylpyrrolidone 25,000 in 46.20 kg purified water is sprayed continuously onto the fluidised bed while drying by heating the air stream of the fluidised bed. At the end of the process 1.76 kg magnesium stearate is sucked into the granulator and mixed with the granules by maintaining the fluidised bed. The resulting granulate is pressed into tablet cores by compression using a rotary tablet press.

For tablets comprising 1 mg of estradiol, 2.22464 kg of hydroxypropylmethylcellulose and 0.44528 macrogol 6000 are dissolved in 14.67 kg purified water. 0.44528 kg talc, 1.25906 kg titanium dioxide and 0.02575 kg ferric oxide pigment are suspended in 10.26 kg purified water with stirring and homogenised. The solution and suspension are combined and used to coat the tablet cores by continuous application of the coating suspension in a coater. For tablets comprising 2 or 3 mg of estradiol, the specific weights of the reagents for the preparation of the coating can easily be calculated.

An alternative formulation for the coatings of 1 mg tablets comprises 2.22464 kg of hydroxypropylmethylcellulose, 0.44528 kg of macrogol 600, 0.44528 kg of talc, 1.17326 of titanium dioxide, 0.07634 kg of ferric oxide pigment, yellow, and 0.03520 kg of ferric oxide pigment, red. A possible formulation for the coatings of 2 mg tablets comprises of 2.22464 kg of hydroxypropylmethylcellulose, 0.44528 kg of macrogol 600, 0.44528 kg of talc, 1.19636 of titanium dioxide and 0.08844 kg of ferric oxide pigment, red. A possible formulation for the coatings of 3 mg tablets comprises of 2.22464 kg of hydroxypropylmethylcellulose, 0.44528 kg of macrogol 600, 0.44528 kg of talc, 1.25906 of titanium dioxide and 0.02574 kg of ferric oxide pigment, red.

### Example 2: Relative bioavailability

The evaluation of relative bioavailability of estradiol (E2) and drospirenone (DRSP), in an open randomised 2-way cross over study with volunteers. E2/DRSP after treatment with either 2 mg of E2 + 2 mg of DRSP, 2 mg of E2 + 6 mg of DRSP coated tablets p.o versus 2 mg of E2 + 2 mg of DRSP oral solution.

### Example 3: Repeated Dose

The evaluation of repeated dose pharmokinetics (accumulation) and potential interaction between estradiol and drospirenone was performed. This open-label, randomised, intra-individual cross-over study of two dose level combinations with an intervening wash-out phase (4 weeks), and multiple application over 28 days was done with 4 dose combinations. A 4-week observation period was performed after the last dose.
**Treatment A:** 1 mg E2 + 1 mg DRSP, daily, p.o.
**Treatment B:** 1 mg E2 + 4 mg DRSP, daily, p.o.
**Treatment C:** 2 mg E2 + 1 mg DRSP, daily, p.o.
**Treatment D:** 2 mg E2 + 4 mg DRSP, daily, p.o.

**Assessment:** No statistically significant interaction was observed between the two active ingredients.

### Example 4: Endometrial Protection

Primary Objective: To evaluate the efficacy of thirteen, 28 day cycles of continuous E2-DRSP combinations compared to continuous E2 by analysis of protection against hyperplasia in post-menopausal women.

Secondary Objectives: To evaluate the effect of endometrial morphology, bleeding patterns, metabolic and hemostatic laboratory parameters. Well-being of post-menopausal women as assessed by the Women's Health Questionnaire (WHQ) and The Medical Outcomes Study 36-Item Short-Form Health Survey (SF-36). Effect on the frequency and severity of hot flushes, and on the relief of urogenital symptoms. Drug levels of DRSP and E2. Detailed evaluation of the metabolic parameters in subgroup.

### Synopsis:

Dose Groups: E2 1 mg; E2 1 mg + DRSP 0.5 mg; E2 1 mg + DRSP 1 mg; E2 1 mg + DRSP 2 mg; E2 1 mg + DRSP 3 mg.

Post-menopausal women with or without menopausal symptoms will be assigned 1 of 5 regimens. Endometrial effects will be evaluated by biopsy to determine the incidence of endometrial hyperplasia. Symptoms and bleeding patterns will be evaluated from subject diaries. General safety, and effects on specific biochemical and hematological parameters will be evaluated. A post-menopausal quality of life assessment and evaluation of patient satisfaction will also be made.

Two-hour glucose tolerance tests, and 15-minute insulin tolerance tests were performed at certain sites.

### Data Analysis

Endometrial biopsy at Visit 1 and Final Visit. Bleeding pattern information is recorded in a daily diary throughout the study.

Two analyses will be performed for the primary efficacy variable: i) 2-sided comparison of treatments and ii) 1-sided, within group interval estimation of dose effect.
Frequency tables will be generated for visits at which endometrial biopsies are performed. These tables will display the number and percent of subjects in each endometrial response category for each treatment group (and by centre in case of a treatment-by-centre interaction). Overall and between group comparison of the incidence of hyperplasia among the treatment groups will be performed. This analysis will be intent-to-treat and will test the null hypothesis of no difference in response to treatments (unopposed vs. opposed estradiol) when adjusted for centre.

### Interval estimation of dose response

The probability πₜ will be estimated for each dose of DRSP (t 0.0, 0.5, 1.0, 2.0, 3.0). Additionally, the upper limit of the one-sided 95% confidence interval will be calculated for each πₜ separately. This means that the confidence intervals will be non-simultaneous.

### Example 5: Osteoporosis Prevention

Primary Objective: Bone mineral density of the hip after 104 weeks of treatment.

Secondary Objectives: Bone mineral density (BMD) of the hip after 12, 28, 52, and 80 weeks of treatment. BMD of the lumbar spine, of the midshaft of the radius, of the total body. Effects on parameters for bone metabolism. Bleeding pattern. General safety.

The study will be performed as a double blind, placebo-controlled trial with 240 healthy, post-menopausal women randomly assigned to one of 4 groups of 60 after giving their informed consent.
- The groups will be as follows:: 1 mg E2 + 1 mg DRSP
1 mg E2 + 2 mg DRSP
1 mg E2 + 3 mg DRSP

### Synopsis

Forty osteopenic patients (BMD hip T-score between -1 and -2.5) and 20 non-osteopenic patients should be enrolled in each group. All treatments will be applied daily *per os* during the whole treatment of 2 years without a treatment-free interval. In addition, the patients will be supplied with calcium tablets (500 mg of calcium daily). Measurements of bone mineral density of the hip will be measured on the left side utilising dual-energy x-ray absorptiometry at screening, baseline, and after 12, 28, 52, 80, and 104 weeks of treatment. Biochemical markers of bone remodelling will be measured at intervals.

Serum bone specific alkaline phosphatase, serum N-mid osteocalcin, urinary calcium/creatine (second morning void), and urinary CrossLaps® /creatine (second morning void) will additionally be evaluated.

### Example 6: Menopausal Symptoms

Objective: To demonstrate that the therapeutic efficacy of E2-DRSP regarding menopausal symptoms is superior to placebo

Primary Objective: Hot flushes

Secondary Objectives: Sweating episodes, sleep problems, depressed moods, nervousness, urogenital symptoms (vaginal dryness, pollakisuria, nocturia), bleeding patterns. General safety.

### Synopsis

Double-blind, placebo controlled trial with healthy, post-menopausal women randomly assigned to one of 4 groups
1 mg E2 + 1 mg DRSP
1 mg E2 + 2 mg DRSP
1 mg E2 + 3 mg DRSP
placebo

### Synopsis:

Main inclusion criterion: A minimum of 5 moderate to severe hot flushes per day on at least 7 days of the 2-week pre-treatment period.

The duration of the treatment will be 16 weeks (4 28-day cycles).

Hot flushes will be assessed by means of recording the frequency and severity (mild, moderate and severe) and comparing these between the verum groups and placebo. The patient will record the incidence and severity of the hot flushes and diary cards. In addition, the investigator will ask the patient at each visit about other typical menopausal symptoms (sweating episodes, sleep problems, depressed moods, nervousness, urogenital symptoms. The intensity of the symptoms will be assessed as mild, moderate or severe.

If the patient has an intact uterus, the occurrence of bleeding will be recorded every day during the study on diary cards. The patient will make daily entries in the diary card according to the bleeding intensity definitions given below

| Code | Category | Definition |
|---|---|---|
| 0 | no | no vaginal bleeding |
| 1 | spotting | less than associated with normal menstruation relative to the subject's experience with no need for sanitary protection (except for panty liners) |
| 2 | light | less than associated with normal menstruation relative to the subject's experience with no need sanitary protection |
| 3 | normal | like normal menstruation relative to the subject's experience |
| 4 | heavy | more than normal menstruation relative to the subject's experience |

For urogential symptoms, the following parameter and categories will be used:

| Parameter | Categories |
|---|---|
| vaginal dryness | yes/no/ not applicable |
| increased frequency of urination | no /yes |
| nocturia | no/yes ("yes" means at least 2urinations during 2 or more nights within the preceding week) |

### Data Analysis

For the efficacy parameters, both a valid-case analysis (VCA) and an intention to treat (ITT) analysis will be performed. The primary target variable is the individual relative change (C) in the number of hot flushes. C is defined as (T-B) / B, where T and B are individual means. T is the mean number of hot flushes per week, calculated by the observations during the weeks 3 through 16 of the treatment phase. B is the mean number of hot flushes per week, calculated from the observations of the 2-week pre-treatment phase.

### Example 7: Lipid Profile

Objective: The primary goal of this study is to compare two E2/DRSP treatments and Premique®/Premelle® regarding lipid profile. Lipid profile is generally accepted as the surrogate endpoint to estimate cardiovascular risk.

Primary Objectives: Lipid pattern including HDL cholesterol, LDL cholesterol

Secondary Objectives: Lipid pattern including total cholesterol, triglycerides, HDL2 cholesterol, HDL3 cholesterol, VLDL cholesterol, apolipoproteins (A-1, A-2, B, E), Lp(a); Menopausal symptoms; Bleeding pattern; Endometrial safety; General safety

Design: The study is performed as an open-label, multicentre, comparative study in 300 post-menopausal women randomly assigned to one of 3 groups of 100 after giving their informed consent.

Groups:
1 mg E2 + 2 mg DRSP
1 mg E2 + 3 mg DRSP
Premique®/Premelle®: 0.625 mg conjugated estrogens + 5 mg MPA

All treatments are applied daily orally during the whole treatment period of 2 years without a treatment free interval. Measurements of lipid profile are performed at screening and after 12, 28, 52, and 104 weeks of treatment as well as six weeks after treatment.

## Claims

1. A pharmaceutical composition in the form of an oral dosage form comprising
i) an estrogen with the exception of ethinyl estradiol;
ii) drospirenone in an amount corresponding to a daily dose ranging from 0.25 to 10 mg; and
iii) a pharmaceutically acceptable excipient or carrier,
wherein said drospirenone is in a form having a surface area of more than 10,000 cm²/g.

2. A pharmaceutical composition in the form of an oral dosage form comprising
i) an estrogen with the exception of ethinyl estradiol;
ii) drospirenone in an amount corresponding to a daily dose ranging from 0.25 to 10 mg; and
iii) a pharmaceutically acceptable excipient or carrier,
wherein drosplrenone is in a form having rapid dissolution such that at least 70% of said drospirenone is dissolved within 30 minutes when the composition is subjected to dissolution testing in 900 ml of water at 37°C using USP XXIII Paddle Method II operated at a stirring rate of 50 rpm.

3. A pharmaceutical composition in the form of an oral dosage form comprising
i) an estrogen with the exception of ethinyl estradiol;
ii) micronised drospirenone in an amount corresponding to a daily dose ranging from 0.25 to 10 mg; and
iii) a pharmaceutically acceptable excipient or carrier.

4. The composition according to claim 1 or 2, wherein drospirenone is sprayed from a solution of drospirenone onto particles of an inert carrier.

5. The composition according to any of claims 1 to 4, wherein the estrogen is selected from the group consisting of estradiol, estradiol sulfamates, estradiol valerate, estradiol benzoate, estrone, estriol, estriol succinate, conjugated estrogen and mixtures thereof.

6. The composition according to claim 5, wherein the conjugated estrogen is selected from the group consisting of estrone sulfate, 17β-estradiol sulfate, 17α-estradiol sulfate, equilin sulfate, 17β-dihydroequilin sulfate, 17α-dihydroequilin sulfate, equilenin sulfate, 17β-dihydroequllenin sulfate and 17α-dihydroequilenin sulfate.

7. The composition according to claim 5, wherein the estrogen is selected from the group consisting of estradiol, estradiol sulfamates, estradiol valerate, estradiol benzoate and mixtures thereof.

8. The composition according to claim 7, wherein the estrogen is estradiol.

9. The composition according to any of claims 1 to 8, wherein the estrogen is in micronised form.

10. The composition according to any of claims 1 to 8, wherein drospirenone is in sufficient amounts to protect the endometrium from the adverse effects of estrogen.

11. The composition according to any of claims 1 to 10, wherein drospirenone is in an amount corresponding to a daily dose ranging from about 0.5 to 4.5 mg.

12. The composition according to any of claims 1 to 11, wherein the estrogen is in sufficient amounts to treat diseases, disorders and symptoms associated with deficient endogenous levels of estrogen in women.

13. The composition according to any of claims 1 to 12, wherein the estrogen is in sufficient amounts to treat diseases, disorders and symptoms associated with natural menopause, peri-menopause, post-menopause, hypogonadism, castration or primary ovarian failure in women, said diseases, disorders and symptoms being selected from the group consisting of hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition and osteoporosis.

14. The composition according to any of claims 1 to 13, wherein estradiol is in an amount corresponding to a daily dose ranging from 0.1 to 5 mg.

15. The composition according to any of claims 1-14, wherein the oral dosage form is in the form of a tablet, a capsule or a pill.

16. A pharmaceutical preparation consisting of a number of separately packaged and individually removable daily dosage units placed into a packaging unit and intended for oral administration for a period of at least 21 days, wherein said dally dosage units comprise a combination of estradiol in an amount ranging from about 0.1 to 5 mg and said drospirenone is in an amount ranging from about 0.25 to 6 mg and is in a form having a surface area of more than 10,000 cm²/g.

17. A pharmaceutical preparation consisting of a number of separately packaged and individually removable daily dosage units placed into a packaging unit and intended for oral administration for a period of at least 21 days, wherein said daily dosage units comprise a combination of estradiol in an amount ranging from about 0.1 to 5 mg and said drospirenone is in an amount ranging from about 0.25 to 6 mg and is in a form having rapid dissolution such that at least 70% of said drospirenone is dissolved within 30 minutes when the dosage unit is subjected to dissolution testing in 900 ml of water at 37°C using USP XXIII Paddle Method II operated at a stirring rate of 50 rpm.

18. A pharmaceutical preparation consisting of a number of separately packaged and individually removable daily dosage units placed into a packaging unit and intended for oral administration for a period of at least 21 days, wherein said daily dosage units comprise a combination of estradiol in an amount ranging from about 0.1 to 5 mg and drospirenone in an amount ranging from about 0.25 to 6 mg and is in micronised form.

19. The preparation according to any of claims 16-18, wherein said oral administration is for a period of 28 days.

20. The preparation according to any of claims 16-18, intended for oral administration for a period of 28 days, wherein
at least 21 of said dosage units comprise estradiol in an amount ranging from about 0.1 to 5 mg and drospirenone in an amount ranging from about 0.25 to 6 mg; and
no further than 7 dosage units comprise a blank, a placebo or estradiol in an amount ranging from about 0.1 to 5 mg.

21. The preparation according to any of claims 16-18, intended for oral administration for a period of at least 28 consecutive days, wherein
at least 10 said daily dosage units comprise estradiol in an amount ranging from about 0.1 to 5 mg;
at least 10 said daily dosage units comprises a combination of estradiol in an amount ranging from about 0.1 to 5 mg and drospirenone in an amount ranging from about 0.25 to 6 mg; and
no more than 8 of said daily dosage units comprise a placebo or a blank.

22. The preparation according to any of claims 16-18, intended for oral administration for a period of at least 28 consecutive days, wherein
at least 10 said daily dosage units comprise estradiol in an amount ranging from about 0.1 to 5 mg;
at least 10 said daily dosage units comprises a combination of estradiol in an amount ranging from about 0.1 to 5 mg and drospirenone in an amount ranging from about 0.25 to 6 mg; and
no further than 8 of said daily dosage units comprise estradiol in an amount ranging from about 0.1 to 5 mg.

23. The preparation according to any of claims 16-18, wherein said oral administration is for a period of 21 to 30 consecutive days,
wherein 10 to 15 said daily dosage units comprise a combination of estradiol in an amount ranging from about 0.1 to 5 mg and drospirenone in an amount ranging from about 0.25 to 6 mg; and
further 10 to 15 said daily dosage units comprise estradiol in an amount ranging from about 0.1 to 5 mg.

24. The preparation according to any of claims 16-23, wherein the amount of estradiol in said dosage units varies according to the day of said period of at least 21 days.

25. The preparation according to any of claims 16-23, wherein the amount of estradiol in said dosage units is the same in each of the dosage unit in the packaging unit.

26. The preparation according to any of claims 16-25, wherein the amount of drospirenone in said dosage units is the same in each of the dosage unit in the packaging unit.

27. The preparation according to any of claims 16-26, wherein drospirenone is sprayed from a solution of drospirenone onto particles of an inert carrier.

28. The preparation according to any of claims 16-27, wherein said estradiol is in micronised form or sprayed from a solution onto particles of inert carrier.

29. The preparation according to any of claims 16-18 and 22-28, wherein the number of daily dosage units is at least 21 or a multiple of 21.

30. The preparation according to claim 29, wherein the number of daily dosage units is a 2 to 12 multiple of 21.

31. The preparation according to any of claims 16-28, wherein the number of daily dosage units is 28 or a multiple of 28.

32. The preparation according to claim 31, wherein the number of daily dosage units is a 2 to 12 multiple of 28.

33. The preparation according to claim 32, wherein the number of daily dosage units is a 2 to 6 multiple of 28.

34. The preparation according to any of claims 16-33, wherein the oral dosage form is in the form of a tablet, capsule or pill.

35. Use of a combination of an estrogen and drospirenone for the preparation of a medicament intended for oral administration and for treating diseases, disorders and symptoms associated with natural menopause, peri-menopause, post-menopause, hypogonadism, castration or primary ovarian failure in women,
said diseases, disorders and symptoms being selected from the group consisting of hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition and osteoporosis, wherein
the amount of drospirenone is sufficient to protect the endometrium from the adverse effects of an estrogen and said drospirenone is in a form having a surface area of more than 10,000 cm²/g

36. Use of a combination of an estrogen and drospirenone for the preparation of a medicament intended for oral administration and for treating diseases, disorders and symptoms associated with natural menopause, peri-menopause, post-menopause, hypogonadism, castration or primary ovarian failure in women,
said diseases, disorders and symptoms being selected from the group consisting of hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition and osteoporosis, wherein
the amount of drospirenone is sufficient to protect the endometrium from the adverse effects of an estrogen and wherein drospirenone is in a form having rapid dissolution such that at least 70% of said drospirenone is dissolved within 30 minutes when the medicament is subjected to dissolution testing in 900 ml of water at 37°C using USP XXIII Paddle Method II operated at a stirring rate of 50 rpm.

37. Use of a combination of an estrogen and drospirenone for the preparation of a medicament intended for oral administration and for treating diseases, disorders and symptoms associated with natural menopause, peri-menopause, post-menopause, hypogonadism, castration or primary ovarian failure in women,
said diseases, disorders and symptoms being selected from the group consisting of hot flushes, sweating attacks, palpitations, sleep disorders, mood changes, nervousness, anxiety, poor memory, loss of confidence, loss of libido, poor concentration, diminished energy, diminished drive, irritability, urogenital atrophy, atrophy of the breasts, cardiovascular disease, changes in hair distribution, thickness of hair, changes in skin condition and osteoporosis, wherein
the amount of drospirenone is sufficient to protect the endometrium from the adverse effects of an estrogen and said drospirenone is in micronised form.

38. The use according to claim 35 or 36, wherein drospirenone is sprayed from a solution of drospirenone onto particles of an inert carrier.

39. The use according to any of claims 35 to 38, wherein the estrogen is in micronised form.

40. The use according to claim 39, wherein said micronised estrogen is such that 100% of the particles have a diameter of ≤ 15.0 µm.

41. The use according to claim 40, wherein said micronised estrogen is such that 95% of the particles have a diameter of ≤ 10.0 µm.

42. The use according to any of claims 35 to 41, wherein the estrogen is in an amount sufficient for the treatment of diseases, disorders and symptoms selected from the group consisting of hot flushes, sweating attacks, palpitations, sleep disorders, mood changes, nervousness, anxiety, urogenital atrophy and atrophy of the breasts or for the prevention or management of osteoporosis.

43. The use according to claim 42, wherein the estrogen is in an amount sufficient for the treatment of hot flushes, sweating attacks or palpitations.

44. The use according to any of claims 35 to 43, wherein the estrogen is selected from the group consisting of estradiol, estradiol sulfamates, estradiol valerate, estradiol benzoate, ethinyl estradiol, estrone, estriol, estriol succinate and conjugated estrogen.

45. The use according to claim 44, wherein the conjugated estrogen is selected from the group consisting of estrone sulfate, 17β-estradiol sulfate, 17α-estradiol sulfate, equilin sulfate, 17β-dihydroequilin sulfate, 17α-dihydroequilln sulfate, equilenin sulfate, 17β-dihydroequilenin sulfate and 17α-dihydroequilenin sulfate.

46. The use according to claim 44, wherein the estrogen is selected from the group consisting of estradiol, estradiol sulfamates, estradiol valerate, estradiol benzoate and mixtures thereof.

47. The use according to claim 46, wherein the estrogen is estradiol.

48. The use according to any of claims 35-47, wherein the amount of drospirenone corresponds to a daily dose ranging from 0.5 to 4.5 mg.

49. The use according to claim 47, wherein estradiol is in amount corresponding to a daily dose ranging from 0.1 to 5 mg.

50. The use according to any of claims 35-49, wherein the oral dosage form is in the form of a tablet, capsule or pill.

51. The use according to any of claims 35 to 49, wherein the medicament is in the form a of a number of separately packaged and individually removable dosage units placed in a packaging unit and intended for oral administration for a period of at least 21 days, preferably for at least 28 days.

52. The use according to claim 51, wherein the dose of estrogen is the same in each of the dosage units in the packaging unit.

53. The use according to claim 51 or 52, wherein the dose of drospirenone is the same in each of the dosage units in the packaging unit.

54. The use according to any of claims 51-53, wherein the individually removable dosage units is in the form of a composition as defined in any of claims 1 to 15.

55. The use according to claim 54, wherein the medicament is in the form of a number of separately packaged and individually removable dosage units placed in a packaging unit as defined in any of daims 16-34.

## Patentansprüche

1. Pharmazeutische Zusammensetzung in Form einer oralen Dosierform, umfassend:
i) ein Estrogen mit Ausnahme von Ethinylestradiol;
ii) Drospirencn in einer Menge entsprechend einer Tagesdosis im Bereich von 0,25 bis 10 mg und
iii) einen pharmazeutisch annehmbaren Hilfsstoff oder Träger,
wobei das Drospirenon in einer Form mit einer Oberfläche von mehr als 10.000 cm²/g vorliegt.

2. Pharmazeutische Zusammensetzung in Form einer oralen Dosierform, umfassend:
i) ein Estrogen mit Ausnahme von Ethinylestradiol;
ii) Drospirenon in einer Menge entsprechend einer Tagesdosis im Bereich von 0,25 bis 10 mg und
iii) einen pharmazeutisch annehmbaren Hilfsstoff oder Träger,
wobei Drospirenon in einer Form mit rascher Auflösung vorliegt, so dass mindestens 70 % des Drospirenons innerhalb von 30 Minuten aufgelöst sind, wenn die Zusammensetzung Auflösungstests in 900 ml Wasser bei 37 °C unter Verwendung des Verfahrens USP XXIII Paddle Method II unterzogen wird, das mit einer Rührgeschwindigkeit von 50 UpM arbeitet.

3. Pharmazeutische Zusammensetzung in Form einer oralen Dosierform, umfassend:
i) ein Estrogen mit Ausnahme von Ethinylestradiol;
ii) mikronisiertes Drospirenon in einer Menge entsprechend einer Tagesdosis im Bereich von 0,25 bis 10 mg und
iii) einen pharmazeutisch annehmbaren Hilfsstoff oder Träger.

4. Zusammensetzung nach Anspruch 1 oder 2, wobei Drospirenon aus einer Lösung von Drospirenon auf Partikel eines inerten Trägers gesprüht ist.

5. Zusammensetzung nach einem der Ansprüche 1 bis 4, wobei das Estrogen aus der Gruppe bestehend aus Estradiol, Estradiolsulfamaten, Estradiolvalerat, Estradiolbenzoat, Estron, Estriol, Estriolsuccinat, konjugiertem Estrogen oder Mischungen davon ausgewählt ist.

6. Zusammensetzung nach Anspruch 5, wobei das konjugierte Estrogen aus der Gruppe bestehend aus Estronsulfat, 17β-Estradiolsulfat, 17α-Estradiolsulfat, Equilinsulfat, 17β-Dihydroequilinsulfat, 17α-Dihydroequilinsulfat, Equileninsulfat, 17β-Dihydroequileninsulfat und 17α-Dihydroequileninsulfat ausgewählt ist.

7. Zusammensetzung nach Anspruch 5, wobei das Estrogen aus der Gruppe bestehend aus Estradiol, Estradiolsulfamaten, Estradiolvalerat, Estradiolbenzoat und Mischungen davon ausgewählt ist.

8. Zusammensetzung nach Anspruch 7, wobei das Estrogen Estradiol ist.

9. Zusammensetzung nach einem der Ansprüche 1 bis 8, wobei das Estrogen in mikronisierter Form vorliegt.

10. Zusammensetzung nach einem der Ansprüche 1 bis 8, wobei Drospirenon in ausreichenden Mengen vorliegt, um das Endometrium vor den nachteiligen Wirkungen von Estrogen zu schützen.

11. Zusammensetzung nach einem der Ansprüche 1 bis 10, wobei Drospirenon in einer Menge entsprechend einer Tagesdosis im Bereich von etwa 0,5 bis 4,5 vorliegt.

12. Zusammensetzung nach einem der Ansprüche 1 bis 11, wobei das Estrogen in ausreichenden Mengen vorliegt, um Erkrankungen, Störungen und Symptome zu behandeln, die mit unzureichenden endogenen Estrogenniveaus bei Frauen in Verbindung stehen.

13. Zusammensetzung nach einem der Ansprüche 1 bis 12, wobei das Estrogen in ausreichenden Mengen vorliegt, um Erkrankungen, Störungen und Symptome zu behandeln, die mit natürlicher Menopause, Perimenopause, Postmenopause, Hypogonadismus, Kastration oder primärem Ausfall des Eierstocks bei Frauen in Verbindung stehen, wobei die Erkrankungen, Störungen und Symptome aus der Gruppe bestehend aus Hitzewallungen, Schweißausbrüchen, Herzklopfen, Schlafstörungen, Stimmungsschwankungen, Nervosität, Ängstlichkeit, schlechtem Gedächtnis, fehlendem Selbstvertrauen, Libidoverlust, schlechter Konzentration, Energiemangel, Antriebsschwäche, Reizbarkeit, Urogenitalatrophie, Atrophie der Brüste, Herz-Kreislauf-Erkrankung, Änderung der Haarverteilung, Haardicke, Änderungen des Hautzustands und Osteoporose ausgewählt sind.

14. Zusammensetzung nach einem der Ansprüche 1 bis 13, wobei Estradiol in einer Menge entsprechend einer Tagesdosis im Bereich von 0,1 bis 5 mg vorliegt.

15. Zusammensetzung nach einem der Ansprüche 1-14, wobei die orale Dosierform in Form einer Tablette, einer Kapsel oder einer Pille vorliegt.

16. Pharmazeutische Zubereitung, die aus einer Anzahl separat verpackter und individuell entfernbarer Tagesdcsiereinheiten besteht, die in einer Verpackungseinheit angeordnet und zur oralen Verabreichung über einen Zeitraum von mindestens 21 Tagen vorgesehen sind, wobei die Tagesdosiereinheiten eine Kombination von Estradiol in einer Menge im Bereich von etwa 0,1 bis 5 mg umfassen und das Drospirenon in einer Menge im Bereich von etwa 0,25 bis 6 mg und in einer Form mit einer Oberfläche von mehr als 10.000 cm²/g vorliegt.

17. Pharmazeutische Zubereitung, die aus einer Anzahl separat verpackter und individuell entfernbarer Tagesdosiereinheiten besteht, die in einer Verpackungseinheit angeordnet und zur oralen Verabreichung über einen Zeitraum von mindestens 21 Tagen vorgesehen sind, wobei die Tagesdosiereinheiten eine Kombination von Estradiol in einer Menge im Bereich von etwa 0,1 bis 5 mg umfassen und das Drospirenon in einer Menge im Bereich von etwa 0,25 bis 6 mg und in einer Form mit rascher Auflösung vorliegt, so dass sich mindestens 70 % des Drospirenons innerhalb von 30 Minuten auflösen, wenn die Dosiereinheit Auflösungstests in 900 ml Wasser bei 37 °C unter Verwendung des Verfahrens USP XXIII Paddle Method II unterzogen wird, das mit einer Rührgeschwindigkeit von 50 UpM arbeitet.

18. Pharmazeutische Zubereitung, die aus einer Anzahl separat verpackter und individuell entfernbarer Tagesdosiereinheiten besteht, die in einer Verpackungseinheit angeordnet und zur oralen Verabreichung über einen Zeitraum von mindestens 21 Tagen vorgesehen sind, wobei die Tagesdosiereinheiten eine Kombination von Estradiol in einer Menge im Bereich von etwa 0,1 bis 5 mg umfassen und das Drospirenon in einer Menge im Bereich von etwa 0,25 bis 6 mg und in mikronisierter Form vorliegt.

19. Zubereitung nach einem der Ansprüche 16-18, wobei die orale Verabreichung für einen Zeitraum von 28 Tagen ist.

20. Zubereitung nach einem der Ansprüche 16-18, die zur oralen Verabreichung über einen Zeitraum von 28 Tagen vorgesehen ist, wobei
mindestens 21 der Dosiereinheiten Estradiol in einer Menge im Bereich von etwa 0,1 bis 5 mg und Drospirenon in einer Menge im Bereich von etwa 0,25 bis 6 mg umfassen; und
nicht mehr als 7 Dosiereinheiten eine Blindprobe, einen Placebo oder Estradiol in einer Menge im Bereich von etwa 0,1 bis 5 mg umfassen.

21. Zubereitung nach einem der Ansprüche 16-18, die zur oralen Verabreichung über einen Zeitraum von 28 aufeinanderfolgenden Tagen vorgesehen ist, wobei
mindestens 10 der Tagesdosiereinheiten Estradiol in einer Menge im Bereich von etwa 0,1 bis 5 mg umfassen;
mindestens 10 der Tagesdosiereinheiten eine Kombination von Estradiol in einer Menge im Bereich von etwa 0,1 bis 5 mg und Drospirenon in einer Menge im Bereich von etwa 0,25 bis 6 mg umfassen; und
nicht mehr als 8 der Tagesdosiereinheiten einen Placebo oder eine Blindprobe umfassen.

22. Zubereitung nach einem der Ansprüche 16-18, die zur oralen Verabreichung über einen Zeitraum von 28 aufeinanderfolgenden Tagen vorgesehen ist, wobei
mindestens 10 der Tagesdosiereinheiten Estradiol in einer Menge im Bereich von etwa 0,1 bis 5 mg umfassen;
mindestens 10 der Tagesdosiereinheiten eine Kombination von Estradiol in einer Menge im Bereich von etwa 0,1 bis 5 mg und Drospirenon in einer Menge im Bereich von etwa 0,25 bis 6 mg umfassen; und
nicht mehr als 8 der Dosiereinheiten Estradiol in einer Menge im Bereich von etwa 0,1 bis 5 mg umfassen.

23. Zubereitung nach einem der Ansprüche 16-18, wobei die orale Verabreichung für einen Zeitraum von 21 bis 30 aufeinanderfolgenden Tagen vorgesehen ist, wobei
10 bis 15 der Tagesdosiereinheiten eine Kombination von Estradiol in einer Menge im Bereich von etwa 0,1 bis 5 mg und Drospirenon in einer Menge im Bereich von etwa 0,25 bis 6 mg umfassen; und
weitere 10 bis 15 der Tagesdosiereinheiten Estradiol in einer Menge im Bereich von etwa 0,1 bis 5 mg umfassen.

24. Zubereitung nach einem der Ansprüche 16-23, wobei die Menge an Estradiol in den Dosiereinheiten gemäß dem Tag des Zeitraums von mindestens 21 Tagen variiert.

25. Zubereitung nach einem der Ansprüche 16-23, wobei die Menge an Estradiol in den Dosiereinheiten in jeder Dosiereinheit in der Verpackungseinheit gleich ist.

26. Zubereitung nach einem der Ansprüche 16-25, wobei die Menge an Drospirenon in den Dosiereinheiten in jeder Dosiereinheit in der Verpackungseinheit gleich ist.

27. Zubereitung nach einem der Ansprüche 16-26, wobei Drospirenon aus einer Lösung von Drospirenon auf Partikel eines inerten Trägers gesprüht ist.

28. Zubereitung nach einem der Ansprüche 16-27, wobei das Estradiol in mikronisierter Form vorliegt oder aus einer Lösung auf Partikel eines inerten Trägers gesprüht ist.

29. Zubereitung nach einem der Ansprüche 16-18 und 22-28, wobei die Anzahl der Tagesdosiereinheiten mindestens 21 oder ein Vielfaches von 21 ist.

30. Zubereitung nach Anspruch 29, wobei die Anzahl der Tagesdosiereinheiten ein 2 bis 12 Vielfaches von 21 ist.

31. Zubereitung nach einem der Ansprüche 16-28, wobei die Anzahl der täglichen Dosiereinheiten 28 oder ein Vielfaches von 28 ist.

32. Zubereitung nach Anspruch 31, wobei die Anzahl der Tagesdosiereinheiten ein 2 bis 12 Vielfaches von 28 ist.

33. Zubereitung nach Anspruch 32, wobei die Anzahl der Tagesdosiereinheiten ein 2 bis 6 Vielfaches von 28 ist.

34. Zubereitung nach einem der Ansprüche 16-33, wobei die orale Dosierform in Form einer Tablette, Kapsel oder Pille vorliegt.

35. Verwendung einer Kombination eines Estrogens und Drospirenon für die Zubereitung eines Medikaments zur oralen Verabreichung und zur Behandlung von Erkrankungen, Störungen und Symptomen, die mit natürlicher Menopause, Perimenopause, Postmenopause, Hypogonadismus, Kastration oder primärem Ausfall des Eierstocks bei Frauen in Verbindung stehen, wobei die Erkrankungen, Störungen und Symptome aus der Gruppe bestehend aus Hitzewallungen, Schweißausbrüchen, Herzklopfen, Schlafstörungen, Stimmungsschwankungen, Nervosität, Ängstlichkeit, schlechtem Gedächtnis, fehlendem Selbstvertrauen, Libidoverlust, schlechter Konzentration, Energiemangel, Antriebsschwäche, Reizbarkeit, Urogenitalatrophie, Atrophie der Brüste, Herz-Kreislauf-Erkrankung, Änderung der Haarverteilung, Haardicke, Änderungen des Hautzustands und Osteoporose ausgewählt sind, wobei
die Menge an Drospirenon ausreichend ist, um das Endometrium vor den nachteiligen Auswirkungen eines Estrogens zu schützen und das Drospirenon in einer Form mit einer Oberfläche von mehr als 10.000 cm²/g vorliegt.

36. Verwendung einer Kombination eines Estrogens und Drospirenon zur Zubereitung eines Medikaments zur oralen Verabreichung und zur Behandlung von Erkrankungen, Störungen und Symptomen, die mit natürlicher Menopause, Perimenopause, Postmenopause, Hypogonadismus, Kastration oder primärem Ausfall des Eierstocks bei Frauen in Verbindung stehen,
wobei die Erkrankungen, Störungen und Symptome aus der Gruppe bestehend aus Hitzewallungen, Schweißausbrüchen, Herzklopfen, Schlafstörungen, Stimmungsschwankungen, Nervosität, Ängstlichkeit, schlechtem Gedächtnis, fehlendem Selbstvertrauen, Libidoverlust, schlechter Konzentration, Energiemangel, Antriebsschwäche, Reizbarkeit, Urogenitalatrophie, Atrophie der Brüste, Herz-Kreislauf-Erkrankung, Änderung der Haarverteilung, Haardicke, Änderungen des Hautzustands und Osteoporose ausgewählt sind, wobei
die Menge an Drospirenon ausreichend ist, um das Endometrium vor den nachteiligen Auswirkungen eines Estrogens zu schützen und das Drospirenon in einer Form mit rascher Auflösung vorliegt, so dass mindestens 70 % des Drospirenons innerhalb von 30 Minuten aufgelöst sind, wenn das Medikament Auflösungstests in 900 ml Wasser bei 37 °C unter Verwendung des Verfahrens USP XXIII Paddle Method II unterzogen wird, das mit einer Rührgeschwindigkeit von 50 UpM arbeitet.

37. Verwendung einer Kombination eines Estrogens und Drospirenon zur Zubereitung eines Medikaments zur oralen Verabreichung und zur Behandlung von Erkrankungen, Störungen und Symptomen, die mit natürlicher Menopause, Perimenopause, Postmenopause, Hypogonadismus, Kastration oder primärem Ausfall des Eierstocks bei Frauen in Verbindung stehen,
wobei die Erkrankungen, Störungen und Symptome aus der Gruppe bestehend aus Hitzewallungen, Schweißausbrüchen, Herzklopfen, Schlafstörungen, Stimmungsschwankungen, Nervosität, Ängstlichkeit, schlechtem Gedächtnis, fehlendem Selbstvertrauen, Libidoverlust, schlechter Konzentration, Energiemangel, Antriebsschwäche, Reizbarkeit, Urogenitalatrophie, Atrophie der Brüste, Herz-Kreislauf-Erkrankung, Änderung der Haarverteilung, Haardicke, Änderungen des Hautzustands und Osteoporose ausgewählt sind, wobei
die Menge an Drospirenon ausreichend ist, um das Endometrium vor den nachteiligen Auswirkungen eines Estrogens zu schützen und das Drospirenon in mikronisierter Form vorliegt.

38. Verwendung nach Anspruch 35 oder 36, wobei Drospirenon aus einer Drospirenonlösung auf Partikel eines inerten Trägers gesprüht ist.

39. Verwendung nach einem der Ansprüche 35 bis 38, wobei das Estrogen in mikronisierter Form vorliegt.

40. Verwendung nach Anspruch 39, wobei das mikronisierte Estrogen derart ist, dass 100 % der Partikel einen Durchmesser ≤ 15,0 µm haben.

41. Verwendung nach Anspruch 40, wobei das mikronisierte Estrogen derart ist, dass 95 % der Partikel einen Durchmesser ≤ 10,0 µm haben.

42. Verwendung nach einem der Ansprüche 35 bis 41, wobei das Estrogen in einer ausreichenden Menge zur Behandlung von Erkrankungen, Störungen und Symptomen ausgewählt aus der Gruppe bestehend aus Hitzewallungen, Schweißausbrüchen, Herzklopfen, Schlafstörungen, Stimmungsschwankungen, Nervosität, Ängstlichkeit, Urogenitalatrophie und Atrophie der Brüste oder zur Verhinderung oder des Managements von Osteoporose vorliegt.

43. Verwendung nach Anspruch 42, wobei das Estrogen in einer ausreichenden Menge zur Behandlung von Hitzewallungen, Schweißausbrüchen oder Herzklopfen vorliegt.

44. Verwendung nach einem der Ansprüche 35 bis 43, wobei das Estrogen aus der Gruppe bestehend aus Estradiol, Estradiolsulfamaten, Estradiolvalerat, Estradiolbenzoat, Ethinylestradiol, Estron, Estriolsuccinat und konjugiertem Estrogen ausgewählt ist.

45. Verwendung nach Anspruch 44, wobei das konjugierte Estrogen aus der Gruppe bestehend aus Estronsulfat, 17β-Estradiolsulfat, 17α-Estradiolsulfat, Equilinsulfat, 17β-Dihydroequilinsulfat, 17α-Dihydroequilinsulfat, Equileninsulfat, 17β-Dihydroequileninsulfat und 17α-Dihydroequileninsulfat ausgewählt ist.

46. Verwendung nach Anspruch 44, wobei das Estrogen aus der Gruppe bestehend aus Estradiol, Estradiolsulfamaten, Estradiolvalerat, Estradiolbenzoat und Mischungen davon ausgewählt ist.

47. Verwendung nach Anspruch 46, wobei das Estrogen Estradiol ist.

48. Verwendung nach einem der Ansprüche 35-47, wobei die Drospirenonmenge einer Tagesdosis im Bereich von 0,5 bis 4,5 mg entspricht.

49. Verwendung nach Anspruch 47, wobei Estradiol in einer Menge entsprechend einer Tagesdosis im Bereich von 0,1 bis 5 mg vorliegt.

50. Verwendung nach einem der Ansprüche 35-49, wobei die orale Dosierform in Form einer Tablette, Kapsel oder Pille vorliegt.

51. Verwendung nach einem der Ansprüche 35 bis 49, wobei das Medikament in Form einer Anzahl separat verpackter und individuell entfernbarer Dosiereinheiten vorliegt, die in einer Verpackungseinheit angeordnet sind und zur oralen Verabreichung für einen Zeitraum von mindestens 21 Tagen, vorzugsweise mindestens 28 Tagen vorgesehen sind.

52. Verwendung nach Anspruch 51, wobei die Estrogendosis in jeder der Dosiereinheiten in der Verpackungseinheit gleich ist.

53. Verwendung nach Anspruch 51 oder 52, wobei die Drospirenondosis in jeder der Dosiereinheiten in der Verpackungseinheit gleich ist.

54. Verwendung nach einem der Ansprüche 51-53, wobei die individuell entfernbaren Dosiereinheiten in Form einer Zusammensetzung wie in einem der Ansprüche 1 bis 15 definiert vorliegen.

55. Verwendung nach Anspruch 54, wobei das Medikament in Form einer Anzahl separat verpackter und individuell entfernbarer Dosiereinheiten vorliegt, die in einer Verpackungseinheit angeordnet sind, wie in einem der Ansprüche 16-34 definiert ist.

## Revendications

1. Composition pharmaceutique sous forme d'une forme de dosage orale comprenant :
i) un oestrogène, à l'exception de l'éthinylestradiol ;
ii) de la drospirénone en une quantité correspondant à une dose journalière allant de 0,25 à 10 mg ; et
iii) un excipient ou un véhicule pharmaceutiquement acceptable,
dans laquelle ladite drospirénone est sous une forme ayant une surface supérieure à 10 000 cm²/g.

2. Composition pharmaceutique sous forme d'une forme de dosage orale comprenant :
i) un oestrogène, à l'exception de l'éthinylestradiol ;
ii) de la drospirénone en une quantité correspondant à une dose journalière allant de 0,25 à 10 mg ; et
iii) un excipient ou un véhicule pharmaceutiquement acceptable,
dans laquelle la drospirénone est sous une forme ayant une solubilisation rapide, telle qu'au moins 70% de ladite drospirénone soit solubilisée dans les 30 minutes lorsque la composition est soumise à un essai de solubilisation dans 900 ml d'eau à 37°C à l'aide d'une Méthode à Pales II USP XXIII fonctionnant à une vitesse d'agitation de 50 rpm.

3. Composition pharmaceutique sous forme d'une forme de dosage orale comprenant :
i) un oestrogène, à l'exception de l'éthinylestradiol ;
ii) de la drospirénone micronisée en une quantité correspondant à une dose journalière allant de 0,25 à 10 mg ; et
iii) un excipient ou un véhicule pharmaceutiquement acceptable.

4. Composition selon la revendication 1 ou 2, dans laquelle la drospirénone est pulvérisée à partir d'une solution de drospirénone sur des particules formées d'un véhicule inerte.

5. Composition selon l'une quelconque des revendications 1 à 4, dans laquelle l'oestrogène est choisi parmi le groupe constitué par l'estradiol, les sulfamates d'estradiol, le valérate d'estradiol, le benzoate d'estradiol, l'estrone, l'estriol, le succinate d'estriol, l'oestrogène conjugué et des mélanges de ceux-ci.

6. Composition selon la revendication 5, dans laquelle l'oestrogène conjugué est choisi parmi le groupe constitué par le sulfate d'estrone, le sulfate de 17β-estradiol, le sulfate de 17α-estradiol, le sulfate d'équiline, le sulfate de 17β-dihydroéquiline, le sulfate de 17α-dihydroéquiline, le sulfate d'équilénine, le sulfate de 17β-dihydroéquilénine et le sulfate de 17α-dihydroéquilénine.

7. Composition selon la revendication 5, dans laquelle l'oestrogène est choisi parmi le groupe constitué par l'estradiol, les sulfamates d'estradiol, le valérate d'estradiol, le benzoate d'estradiol et des mélanges de ceux-ci.

8. Composition selon la revendication 7, dans laquelle l'estrogène est l'estradiol.

9. Composition selon l'une quelconque des revendications 1 à 8, dans laquelle l'oestrogène est sous forme micronisée.

10. Composition selon l'une quelconque des revendications 1 à 8, dans laquelle la drospirénone se trouve en quantités suffisantes pour protéger l'endomètre des effets néfastes de l'oestrogène.

11. Composition selon l'une quelconque des revendications 1 à 10, dans laquelle la drospirénone se trouve en une quantité correspondant à une dose journalière allant d'environ 0,5 à 4,5 mg.

12. Composition selon l'une quelconque des revendications 1 à 11, dans laquelle l'oestrogène se trouve en des quantités suffisantes pour traiter des maladies, des troubles et des symptômes associés à des taux endogènes déficients d'oestrogène chez la femme.

13. Composition selon l'une quelconque des revendications 1 à 12, dans laquelle l'oestrogène se trouve en des quantités suffisantes pour traiter des maladies, des troubles et des symptômes associés à la ménopause naturelle, la péri-ménopause, la post-ménopause, l'hypogonadisme, la castration ou la déficience ovarienne primaire chez la femme, lesdit(e)s maladies, troubles et symptômes étant choisi(e)s parmi le groupe constitué par les bouffées de chaleur, les attaques de sudation, les palpitations, les troubles du sommeil, les changements d'humeur, la nervosité, l'anxiété, les troubles de la mémoire, la perte de confiance, la perte de libido, les troubles de la concentration, la diminution d'énergie, la diminution de l'incitation, l'irritabilité, l'atrophie urogénitale, l'atrophie mammaire, la maladie cardiovasculaire, les modifications de la répartition capillaire, l'épaisseur des cheveux, les modifications de l'état de la peau et l'ostéoporose.

14. Composition selon l'une quelconque des revendications 1 à 13, dans laquelle l'estradiol se trouve en une quantité correspondant à une dose journalière allant de 0,1 à 5 mg.

15. Composition selon l'une quelconque des revendications 1-14, dans laquelle la forme de dosage orale se présente sous la forme d'un comprimé, d'une capsule ou d'une pilule.

16. Préparation pharmaceutique constituée d'un certain nombre d'unités de dosage journalier emballées séparément et pouvant être prélevées individuellement, placées dans une unité d'emballage et prévues pour une administration orale pendant une période d'au moins 21 jours, dans laquelle lesdites unités de dosage journalier comprennent une association d'estradiol en une quantité allant d'environ 0,1 à 5 mg et ladite drospirénone se trouve en une quantité allant d'environ 0,25 à 6 mg et se présente sous une forme ayant une surface supérieure à 10 000 cm²/g.

17. Préparation pharmaceutique constituée d'un certain nombre d'unités de dosage journalier emballées séparément et pouvant être prélevées individuellement, placées dans une unité d'emballage et prévues pour une administration orale pendant une période d'au moins 21 jours, dans laquelle lesdites unités de dosage journalier comprennent une association d'estradiol en une quantité allant d'environ 0,1 à 5 mg et ladite drospirénone se trouve en une quantité allant d'environ 0,25 à 6 mg et elle est sous une forme ayant une solubilisation rapide, telle qu'au moins 70% de ladite drospirénone soit solubilisée dans les 30 minutes lorsque l'unité de dosage est soumise à un essai de solubilisation dans 900 ml d'eau à 37°C à l'aide d'une Méthode à Pales II USP XXIII fonctionnant à une vitesse d'agitation de 50 rpm.

18. Préparation pharmaceutique constituée d'un certain nombre d'unités de dosage journalier emballées séparément et pouvant être prélevées individuellement, placées dans une unité d'emballage et prévues pour une administration orale pendant une période d'au moins 21 jours, dans laquelle lesdites unités de dosage journalier comprennent une association d'estradiol en une quantité allant d'environ 0,1 à 5 mg et de la drospirénone en une quantité allant d'environ 0,25 à 6 mg et qui est sous une forme micronisée.

19. Préparation selon l'une quelconque des revendications 16-18, dans laquelle ladite administration orale est prévue pour une période de 28 jours.

20. Préparation selon l'une quelconque des revendications 16-18, prévue pour une administration orale pendant une période de 28 jours, dans laquelle au moins 21 parmi lesdites unités de dosage comprennent de l'estradiol en une quantité allant d'environ 0,1 à 5 mg et de la drospirénone en une quantité allant d'environ 0,25 à 6 mg ; et
pas plus de 7 unités de dosage comprennent un blanc, un placebo ou de l'estradiol en une quantité allant d'environ 0,1 à 5 mg.

21. Préparation selon l'une quelconque des revendications 16-18, prévue pour une administration orale pendant une période de 28 jours consécutifs, dans laquelle
au moins 10 parmi lesdites unités de dosage journalier comprennent de l'estradiol en une quantité allant d'environ 0,1 à 5 mg ;
au moins 10 parmi lesdites unités de dosage journalier comprennent une association d'estradiol en une quantité allant d'environ 0,1 à 5 mg et de drospirénone en une quantité allant d'environ 0,25 à 6 mg ; et
pas plus de 8 parmi lesdites unités de dosage journalier comprennent un placebo ou un blanc.

22. Préparation selon l'une quelconque des revendications 16-18, prévue pour une administration orale pendant une période d'au moins 28 jours consécutifs, dans laquelle
au moins 10 parmi lesdites unités de dosage journalier comprennent de l'estradiol en une quantité allant d'environ 0,1 à 5 mg ;
au moins 10 parmi lesdites unités de dosage journalier comprennent une association d'estradiol en une quantité allant d'environ 0,1 à 5 mg et de drospirénone en une quantité allant d'environ 0,25 à 6 mg ; et
pas plus de 8 parmi lesdites unités de dosage journalier comprennent de l'estradiol en une quantité allant d'environ 0,1 à 5 mg.

23. Préparation selon l'une quelconque des revendications 16-18, dans laquelle ladite administration orale est prévue pour une période de 21 à 30 jours consécutifs,
dans laquelle de 10 à 15 parmi lesdites unités de dosage journalier comprennent une association d'estradiol en une quantité allant d'environ 0,1 à 5 mg et de drospirénone en une quantité allant d'environ 0,25 à 6 mg ; et
de 10 à 15 unités supplémentaires parmi lesdites unités de dosage journalier comprennent de l'estradiol en une quantité allant d'environ 0,1 à 5 mg.

24. Préparation selon l'une quelconque des revendications 16-23, dans laquelle la quantité d'estradiol dans lesdites unités de dosage varie selon le jour de ladite période d'au moins 21 jours.

25. Préparation selon l'une quelconque des revendications 16-23, dans laquelle la quantité d'estradiol dans lesdites unités de dosage est la même dans chacune des unités de dosage dans l'unité d'emballage.

26. Préparation selon l'une quelconque des revendications 16-25, dans laquelle la quantité de drospirénone dans lesdites unités de dosage est la même dans chacune des unités de dosage dans l'unité d'emballage.

27. Préparation selon l'une quelconque des revendications 16-26, dans laquelle la drospirénone est pulvérisée à partir d'une solution de drospirénone sur des particules formées d'un véhicule inerte.

28. Préparation selon l'une quelconque des revendications 16-27, dans laquelle ledit estradiol est sous une forme micronisée ou est pulvérisé à partir d'une solution sur des particules formées d'un véhicule inerte.

29. Préparation selon l'une quelconque des revendications 16-18 et 22-28, dans laquelle le nombre d'unités de dosage journalier est d'au moins 21 ou un multiple de 21.

30. Préparation selon la revendication 29, dans laquelle le nombre d'unités de dosage journalier est un multiple 2 à 12 de 21.

31. Préparation selon l'une quelconque des revendications 16-28, dans laquelle le nombre d'unités de dosage journalier est de 28 ou un multiple de 28.

32. Préparation selon la revendication 31, dans laquelle le nombre d'unités de dosage journalier est un multiple 2 à 12 de 28.

33. Préparation selon la revendication 32, dans laquelle le nombre d'unités de dosage journalier est un multiple 2 à 6 de 28.

34. Préparation selon l'une quelconque des revendications 16-33, dans laquelle la forme de dosage orale se présente sous la forme d'un comprimé, d'une capsule ou d'une pilule.

35. Utilisation d'une association d'un oestrogène et de drospirénone pour la préparation d'un médicament prévu pour une administration orale et destiné au traitement de maladies, de troubles et de symptômes associés à la ménopause naturelle, la péri-ménopause, la post-ménopause, l'hypogonadisme, la castration ou la déficience ovarienne primaire chez la femme, lesdit(e)s maladies, troubles et symptômes étant choisi(e)s parmi le groupe constitué par les bouffées de chaleur, les attaques de sudation, les palpitations, les troubles du sommeil, les changements d'humeur, la nervosité, l'anxiété, les troubles de la mémoire, la perte de confiance, la perte de libido, les troubles de la concentration, la diminution d'énergie, la diminution de l'incitation, l'irritabilité, l'atrophie urogénitale, l'atrophie mammaire, la maladie cardiovasculaire, les modifications de la répartition capillaire, l'épaisseur des cheveux, les modifications de l'état de la peau et l'ostéoporose, dans laquelle
la quantité de drospirénone est suffisante pour protéger l'endomètre des effets néfastes d'un oestrogène et ladite drospirénone est sous une forme ayant une surface supérieure à 10 000 cm²/g.

36. Utilisation d'une association d'un oestrogène et de drospirénone pour la préparation d'un médicament prévu pour une administration orale et destiné au traitement de maladies, de troubles et de symptômes associés à la ménopause naturelle, la péri-ménopause, la post-ménopause, l'hypogonadisme, la castration ou la déficience ovarienne primaire chez la femme, lesdit(e)s maladies, troubles et symptômes étant choisi(e)s parmi le groupe constitué par les bouffées de chaleur, les attaques de sudation, les palpitations, les troubles du sommeil, les changements d'humeur, la nervosité, l'anxiété, les troubles de la mémoire, la perte de confiance, la perte de libido, les troubles de la concentration, la diminution d'énergie, la diminution de l'incitation, l'irritabilité, l'atrophie urogénitale, l'atrophie mammaire, la maladie cardiovasculaire, les modifications de la répartition capillaire, l'épaisseur des cheveux, les modifications de l'état de la peau et l'ostéoporose, dans laquelle
la quantité de drospirénone est suffisante pour protéger l'endomètre des effets néfastes d'un oestrogène et dans laquelle la drospirénone est sous une forme ayant une solubilisation rapide, telle qu'au moins 70% de ladite drospirénone soit solubilisée dans les 30 minutes lorsque le médicament est soumis à un essai de solubilisation dans 900 ml d'eau à 37 °C à l'aide d'une Méthode à Pales II USP XXIII fonctionnant à une vitesse d'agitation de 50 rpm.

37. Utilisation d'une association d'un oestrogène et de drospirénone pour la préparation d'un médicament prévu pour une administration orale et destiné au traitement de maladies, de troubles et de symptômes associés à la ménopause naturelle, la péri-ménopause, la post-ménopause, l'hypogonadisme, la castration ou la déficience ovarienne primaire chez la femme, lesdit(e)s maladies, troubles et symptômes étant choisi(e)s parmi le groupe constitué par les bouffées de chaleur, les attaques de sudation, les palpitations, les troubles du sommeil, les changements d'humeur, la nervosité, l'anxiété, les troubles de la mémoire, la perte de confiance, la perte de libido, les troubles de la concentration, la diminution d'énergie, la diminution de l'incitation, l'irritabilité, l'atrophie urogénitale, l'atrophie mammaire, la maladie cardiovasculaire, les modifications de la répartition capillaire, l'épaisseur des cheveux, les modifications de l'état de la peau et l'ostéoporose, dans laquelle
la quantité de drospirénone est suffisante pour protéger l'endomètre des effets néfastes d'un oestrogène et ladite drospirénone est sous forme micronisée.

38. Utilisation selon la revendication 35 ou 36, dans laquelle la drospirénone est pulvérisée à partir d'une solution de drospirénone sur des particules formées d'un véhicule inerte.

39. Utilisation selon l'une quelconque des revendications 35 à 38, dans laquelle l'oestrogène est sous forme micronisée.

40. Utilisation selon la revendication 39, dans laquelle ledit oestrogène micronisé est tel que 100% des particules ont un diamètre inférieur ou égal à 15,0 µm.

41. Utilisation selon la revendication 40, dans laquelle ledit oestrogène micronisé est tel que 95% des particules ont un diamètre inférieur ou égal à 10,0 µm.

42. Utilisation selon l'une quelconque des revendications 35 à 41, dans laquelle l'oestrogène est en une quantité suffisante pour le traitement de maladies, de troubles et de symptômes choisi(e)s parmi le groupe constitué par les bouffées de chaleur, les attaques de sudation, les palpitations, les troubles du sommeil, les changements d'humeur, la nervosité, l'anxiété, l'atrophie urogénitale et l'atrophie mammaire ou pour la prévention ou la prise en charge de l'ostéoporose.

43. Utilisation selon la revendication 42, dans laquelle l'oestrogène est en une quantité suffisante pour le traitement des bouffées de chaleur, des attaques de sudation ou des palpitations.

44. Utilisation selon l'une quelconque des revendications 35 à 43, dans laquelle l'oestrogène est choisi parmi le groupe constitué par l'estradiol, les sulfamates d'estradiol, le valérate d'estradiol, le benzoate d'estradiol, l'éthinylestradiol, l'estrone, l'estriol, le succinate d'estriol et l'oestrogène conjugué.

45. Utilisation selon la revendication 44, dans laquelle l'oestrogène conjugué est choisi parmi le groupe constitué par le sulfate d'estrone, le sulfate de 17β-estradiol, le sulfate de 17α-estradiol, le sulfate d'équiline, le sulfate de 17β-dihydroéquiline, le sulfate de 17α-dihydroéquiline, le sulfate d'équilénine, le sulfate de 17β-dihydroéquilénine et le sulfate de 17α-dihydroéquilénine.

46. Utilisation selon la revendication 44, dans laquelle l'oestrogène est choisi parmi le groupe constitué par l'estradiol, les sulfamates d'estradiol, le valérate d'estradiol, le benzoate d'estradiol et des mélanges de ceux-ci.

47. Utilisation selon la revendication 46, dans laquelle l'estrogène est l'estradiol.

48. Utilisation selon l'une quelconque des revendications 35-47, dans laquelle la quantité de drospirénone correspond à une dose journalière allant de 0,5 à 4,5 mg.

49. Utilisation selon la revendication 47, dans laquelle l'estradiol se trouve en une quantité correspondant à une dose journalière allant de 0,1 à 5 mg.

50. Utilisation selon l'une quelconque des revendications 35-49, dans laquelle la forme de dosage orale se présente sous la forme d'un comprimé, d'une capsule ou d'une pilule.

51. Utilisation selon l'une quelconque des revendications 35 à 49, dans laquelle le médicament est sous la forme d'un certain nombre d'unités de dosage emballées séparément et pouvant être prélevées individuellement, placées dans une unité d'emballage et prévues pour une administration orale pendant une période d'au moins 21 jours, préférablement d'au moins 28 jours.

52. Utilisation selon la revendication 51, dans laquelle la dose d'oestrogène est la même dans chacune des unités de dosage dans l'unité d'emballage.

53. Utilisation selon la revendication 51 ou 52, dans laquelle la dose de drospirénone est la même dans chacune des unités de dosage dans l'unité d'emballage.

54. Utilisation selon l'une quelconque des revendications 51-53, dans laquelle les unités de dosage pouvant être prélevées individuellement sont sous la forme d'une composition telle que définie selon l'une quelconque des revendications 1 à 15.

55. Utilisation selon la revendication 54, dans laquelle le médicament est sous la forme d'un certain nombre d'unités de dosage emballées séparément et pouvant être prélevées individuellement, placées dans une unité d'emballage, telles que définies selon l'une quelconque des revendications 16-34.
